# EUROPEAN PATENT SPECIFICATION

(11) **EP 3 819 289 B1**
(45) Date of publication and mention of the grant of the patent: **24.08.2022**
(21) Application number: 19815317.3
(22) Date of filing: 20.07.2019
(51) Int. Cl.: C07C 253/32, C07C 255/46, C07C 233/52, C07C 231/12, C07C 57/145, C07C 253/30, C07C 253/34

(54) **CIS-PARA-SUBSTITUTED CYCLOHEXYLAMINO NITRILE SALT AND PREPARATION METHOD THEREFOR**
CIS-PARA-SUBSTITUIERTE CYCLOHEXYLAMINONITRILSALZE UND VERFAHREN ZU IHRER HERSTELLUNG
SEL DE CYCLOHEXYLAMINO NITRILE À SUSBTITUTION CIS-PARA ET SON PROCÉDÉ DE PRÉPARATION

(30) Priority: 03.10.2018 CN 201811163905
(43) Date of publication of application: 12.05.2021
(73) Proprietor: Hebei Lansheng Biotech Co., Ltd, Hebei 052260 (CN); Hebei Lanrun Plant Protection Technology Co., Ltd., Cangzhou, Hebei 061000 (CN)
(72) Inventor: DONG, Zhipeng, Shijiazhuang, Hebei 052260 (CN); GUO, Qingchun, Shijiazhuang, Hebei 052260 (CN); LIN, Bin, Shijiazhuang, Hebei 052260 (CN); YUAN, Ligang, Shijiazhuang, Hebei 052260 (CN); GUO, Hongyong, Shijiazhuang, Hebei 052260 (CN); ZHANG, Zhihu, Shijiazhuang, Hebei 052260 (CN); XU, Feng, Shijiazhuang, Hebei 052260 (CN); WANG, Shihua, Shijiazhuang, Hebei 052260 (CN); SONG, Haiwen, Shijiazhuang, Hebei 052260 (CN); LIU, Hailong, Shijiazhuang, Hebei 052260 (CN); LI, Longqiang, Shijiazhuang, Hebei 052260 (CN); CUI, Debao, Shijiazhuang, Hebei 052260 (CN); ZHANG, Song, Shijiazhuang, Hebei 052260 (CN); ZHAO, Xiaodong, Shijiazhuang, Hebei 052260 (CN); WU, Zhichao, Shijiazhuang, Hebei 052260 (CN); WANG, Xuhao, Shijiazhuang, Hebei 052260 (CN)
(74) Representative: karo IP
(86) International application number: PCT/CN2019/096930
(87) International publication number: WO 2019/233498

(56) References cited:
- CN-A- 103 270 020
- CN-A- 105 777 581

## Description

### Technical field

The present invention relates to a cis-para-substituted cyclohexylaminonitrile salt and a preparation method therefor, the use of the salt as an intermediate for the preparation of a pesticide, and a method for preparing a pesticide using the salt as an intermediate.

### Background technique

Chinese patent CN103270020B discloses the cis-amino-4-alkoxycyclohexane carbonitrile salt represented by the following formula, 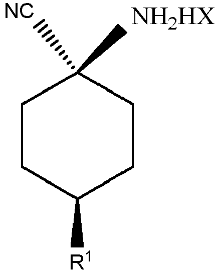 wherein HX is hydrochloric acid, sulfuric acid, phosphoric acid, formic acid, acetic acid, p-toluenesulfonic acid or methanesulfonic acid, and R¹ is alkoxy.

However, the inventor of the present application found that the three inorganic acids of hydrochloric acid, sulfuric acid, and phosphoric acid used in CN103270020B are not selective, and the cis-isomer compound cannot be selectively crystallized and separated by the salt formation of these acids with amino-4-alkoxycyclohexanecarbonitrile; the salt formed by acetic acid and the above cyclohexanecarbonitrile compound cannot be crystallized; the selectivity of p-toluenesulfonic acid and methanesulfonic acid is poor, which is not conducive to industrial production; the salt formed by formic acid and the above cyclohexanecarbonitrile compound is easy to agglomerate, has poor stability, and is easy to decompose, furthermore, the color of the salt becomes darker and the melting point decreases during storage, which seriously affects the subsequent use of the product.

### Summary of the invention

In view of the above problems, the inventor of the present application has conducted in-depth research and found that maleic acid and hydroxy-acetic acid can separate the para-substituted cyclohexylaminonitrile compounds with good selectivity, and the separated cis-isomer salt compound is stable. The invention of this application is completed on the basis of the above findings.

Specifically, the present invention provides:
(1) The cis-para-substituted cyclohexylaminonitrile salt represented by the following formula (I), 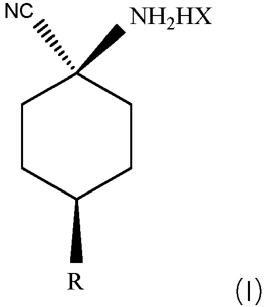 In the above formula (I), the para-substituent R is C₁₋₁₀ alkyl or alkyloxy, C₁₋₁₀ alkenyl or alkenyloxy, C₁₋₁₀ alkynyl or alkynyloxy, C₁₋₁₀ cycloalkyl or cycloalkyloxy, C₁₋₁₀ heterocycloalkyl or heterocycloalkyloxy containing 1-2 heteroatoms selected from O and N;
   HX is hydroxy-acetic acid or maleic acid.
(2) The cis-para-substituted cyclohexylaminonitrile salt according to (1) above, wherein R is preferably the aforementioned groups having 1 to 6 carbon atoms.
(3) The cis-para-substituted cyclohexylaminonitrile salt according to (1) or (2) above, wherein R is C₁₋₆ alkyl or alkyloxy, C₁₋₆ cycloalkyl or cycloalkyloxy Group, C₁₋₆ heterocycloalkyl or heterocycloalkyloxy containing 1-2 heteroatoms selected from O and N.
(4) The cis-para-substituted cyclohexylaminonitrile salt according to any one of (1) to (3) above, wherein R is C₁₋₆ alkyl or alkyloxy.
(5) The cis-para-substituted cyclohexylaminonitrile salt according to any one of (1) to (4) above, wherein R is methyl or methoxy.
(6) The cis-para-substituted cyclohexylaminonitrile salt according to any one of (1) to (5) above, wherein R is methoxy.
(7) The cis-para-substituted cyclohexylaminonitrile salt according to any one of (1) to (6) above, wherein HX is maleic acid.
(8) A preparation method for the cis-para-substituted cyclohexylaminonitrile salt according to any one of (1) to (7) above, which comprises adding maleic acid or hydroxy-acetic acid into the organic solvent solution of the cis/trans para-substituted cyclohexylaminonitrile compound, stirring and crystallizing, and then separating by filtration or the like to obtain the compound of formula (I).
(9) The preparation method according to (8) above, wherein the organic solvent is one selected from methyl *tert*-butyl ether, ethyl acetate and isopropyl acetate, or a mixture thereof.
(10) The preparation method according to (8) or (9) above, wherein the cooling is cooling to 1-10°C.
(11) The preparation method according to any one of (8) to (10) above, relative to the cis/trans para-substituted cyclohexylaminonitrile compound, the added amount of maleic acid or hydroxy-acetic acid is 0.3-0.55 mole times, preferably 0.3-0.5 mole times.
(12) The preparation method according to any one of (8) to (11) above, which further comprises recycling the mother liquor obtained from crystallization and filtration in the preparation method.
(13) A preparation method for the compound (II), wherein the cis-para-substituted cyclohexylaminonitrile salt of formula (I) according to any one of (1)-(7) above is used as a raw material, the salt compound reacts with 2,5-dimethylphenylacetyl chloride in the presence of a diluent and an acid binding agent to obtain compound (II). 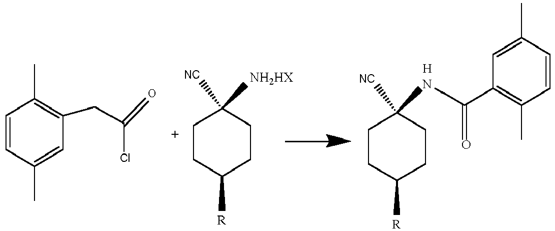 In the above formula, HX and R are the same as defined above.
(14) The preparation method according to (13) above, wherein the diluent is water and any inert solvent that is not miscible with water. The inert solvent is preferably one of toluene, xylene, dichloromethane, dichloroethane, ethyl acetate, isopropyl acetate, or a mixture thereof. The ratio of water to the inert solvent is preferably 1-5:5-1.
(15) The preparation method according to (13) or (14) above, wherein the acid binding agent is alkali metal carbonate or alkali metal bicarbonate or a mixture thereof.
(16) The preparation method according to any one of (13) to (15) above, wherein the reaction temperature is 0-30°C, preferably 0-15°C.
(17) The use of the cis-para-substituted cyclohexylaminonitrile salt of formula (I) according to any one of (1) to (7) above as an intermediate for the preparation of a pesticide, such as spirotetramat.

### Detailed description of the invention

One aspect of the present invention provides a new cis-para-substituted cyclohexylaminonitrile salt represented by the following formula (I):

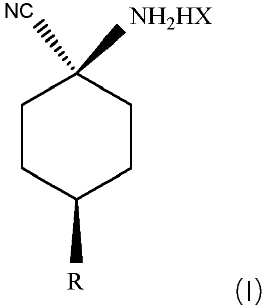

In the above formula (I), R is C₁₋₁₀ alkyl or alkyloxy, C₁₋₁₀ alkenyl or alkenyloxy, C₁₋₁₀ alkynyl or alkynyloxy, C₁₋₁₀ cycloalkyl or cycloalkyloxy, C₁₋₁₀ heterocycloalkyl or heterocycloalkyloxy containing 1-2 heteroatoms selected from O and N; R is preferably the above groups with 1 to 6 carbon atoms, more preferably C₁₋₆ alkyl or alkyloxy, C₁₋₆ cycloalkyl or cycloalkyloxy, C₁₋₆ heterocycloalkyl or heterocycloalkyloxy containing 1-2 heteroatoms selected from O and N, further more preferably C₁₋₆ alkyl or alkyloxy, particularly preferably methyl or methoxy.

HX is hydroxy-acetic acid or maleic acid. These two acids and the para-substituted cyclohexylaminonitrile form a salt with high stability. Among them, maleic acid is more preferred.

The second aspect of the present invention provides a method for preparing the above compound of formula (I), which comprises the following steps: adding maleic acid or hydroxy-acetic acid to an organic solvent solution of a cis/trans para-substituted cyclohexylaminonitrile compound, stirring and crystallizing, and then separating by filtration or the like to obtain the compound of formula (I).

The cis/trans para-substituted cyclohexylaminonitrile compound used in the above preparation method of the present invention can be synthesized by, for example, the well-known Strecker reaction, or can also be synthesized by a known method disclosed in, for example, Chinese Patent Document CN103270020B.

The organic solvent used in the above preparation method of the present invention includes, but is not limited to, methyl tert-butyl ether, ethyl acetate, isopropyl acetate and the like. The above organic solvents may be used alone or in combination of two or more.

The amount of the organic solvent used is preferably 1-10 times by mass, more preferably 2-5 times by mass, and still more preferably 3-4 times by mass relative to the cis/trans para-substituted cyclohexylaminonitrile compound.

The amount of maleic acid or hydroxy-acetic acid used is preferably 0.3-0.55 mole times, more preferably 0.3-0.5 mole times relative to the cis/trans para-substituted cyclohexylaminonitrile compound.

In the above preparation method, the temperature can be lowered by, for example, ice-water bath cooling, preferably to 1-10°C, more preferably to 5-10°C.

In the above preparation method, after crystallization, the precipitated target cis-isomer product is separated by means such as filtration.

The third aspect of the present invention provides a method for preparing compound (II) via the following reaction route in the presence of a diluent and an acid binding agent, wherein the cis-para-substituted cyclohexylaminonitrile salt of formula (I) above is used as a raw material.

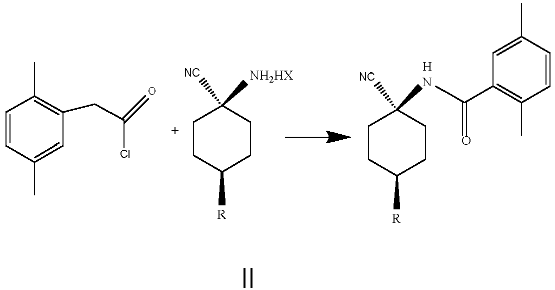

In the above formula, HX and R are the same as defined above.

The above diluent is water and any inert solvent that is not miscible with water. The inert solvent is preferably one of toluene, xylene, dichloromethane, dichloroethane, ethyl acetate, isopropyl acetate, or a mixture thereof. The ratio of water to the inert solvent is preferably 1-5:5-1.

In the present invention, in the above reaction, alkali metal carbonate or alkali metal bicarbonate or a mixture thereof is used as an acid binding agent, whereas in the past, a tertiary amine such as triethylamine was often used as an acid binding agent. However, in industrial production, organic amines such as triethylamine are irritating and dangerous, requiring high-cost treatment and recovery steps. In addition, organic amines such as triethylamine have strong basicity and easily cause hydrolysis of amino nitrile compounds. The inventors of the present application have discovered through research that using alkali metal carbonate or alkali metal bicarbonate or a mixture thereof, compound II can be obtained in high yield.

The above reaction is preferably carried out at about 0-30°C, more preferably at about 0-15°C.

### Examples

### Example 1 cis-p-methoxycyclohexylaminonitrile maleate

Added 600 g of ethyl acetate solution of cis/trans-p-methoxycyclohexyl aminonitrile (cis: trans=55:45) into a three-necked flask, and added 39.2 g of maleic acid (0.34 mole times) at 25-30°C, stirrd for 8-12 hours, then cooled to 5-10°C in an ice-water bath to crystallize. The precipitated maleate is filtered and rinsed with an appropriate amount of ethyl acetate. After vacuum drying at 35-40°C, white cis-p-methoxycyclohexylaminonitrile maleate crystals (75.7g, cis/trans ratio=95.5:4.5) were obtained.

### Example 2 cis-p-methoxycyclohexylaminonitrile maleate

Added 600 g of ethyl acetate solution of cis/trans-p-methoxycyclohexyl aminonitrile (cis: trans=55:45) into a three-necked flask, and added 52.2 g of maleic acid (0.45 mole times) at 25-30°C, stirred for 8-12 hours, then cooled to 5-10°C in an ice-water bath to crystallize. The precipitated maleate is filtered and rinsed with an appropriate amount of ethyl acetate. After vacuum drying at 35-40°C, white cis-p-methoxycyclohexylaminonitrile maleate crystals (108.1g, cis/trans ratio=96.1:3.9) were obtained.

### Example 3 cis-p-methoxycyclohexylaminonitrile hydroxy-acetate

Added 600 g of ethyl acetate solution of cis/trans-p-methoxycyclohexyl aminonitrile (cis: trans=55:45) into a three-necked flask, and added 34.2 hydroxy-acetic acid (0.45 mole times) at 25-30°C , stirred for 8-12 hours, then cooled to 5-10°C in an ice-water bath to crystallize. The precipitated hydroxy-acetate is filtered and rinsed with an appropriate amount of ethyl acetate. After vacuum drying at 35-40 ° C, white cis-p-methoxycyclohexylaminonitrile hydroxy-acetate crystals (92.1g, cis/trans ratio=94:6) were obtained.

### Comparative example 1 cis-p-methoxycyclohexyl carbamate

Added 600 g of ethyl acetate solution of cis/trans-p-methoxycyclohexyl aminonitrile (cis: trans=55:45) into a three-necked flask, and added 20.7 g of formic acid (0.45 mole times) at 25-30°C. When half the amount of formic acid was added, a large amount of solids precipitated and agglomerated. Kept stirring to disperse, and cooled to 5-10°C in an ice water bath to crystallize. The precipitated formate was filtered and rinsed with an appropriate amount of ethyl acetate. After vacuum drying at 35-40°C, white cis-p-methoxycyclohexyl aminonitrile formate crystals (64.1g, cis/trans ratio=95:5) were obtained.

### Comparative example 2

Added 600 g of ethyl acetate solution of cis/trans-p-methoxycyclohexyl aminonitrile (cis: trans=55:45) into a three-necked flask, and added 27.0 g of acetic acid (0.45 mole times) at 25-30°C , stirred for 8-12 hours, then cooled to 5-10°C in an ice-water bath to crystallize overnight, but no crystal was precipitated.

### Comparative example 3

Added 600 g of ethyl acetate solution of cis/trans-p-methoxycyclohexyl aminonitrile (cis: trans=55:45) into a three-necked flask, and added 77.5 g of p-toluenesulfonic acid ( 0.45 mole times) at 25-30°C, stirred for 8-12 hours, then cooled to 5-10°C in an ice-water bath for crystallization, filtered the precipitated p-toluenesulfonate and rinsed with an appropriate amount of ethyl acetate. After drying, white cis/trans-p-methoxycyclohexylaminonitrile p-toluenesulfonate crystals (137.0g, cis/trans ratio 55:45) were obtained.

### Example 4 Product stability comparison test

Took 20g of the samples prepared in example 1, example 2, and comparative example 1, respectively, and stored them in a sample room at 25-30°C and a humidity of 40-45%. Samples were taken on the 7th, 14th day, 1 month, and 3 months for appearance and content inspection.

As results, the products of examples 1 and 2 remained white in each test on the 7, 14 days, 1 month, and 3 months, and the content was above 90%. On the other hand, the content of the product of comparative example 1 decreased to 86.2% on the 14th day, the color changed to gray and the content decreased to 62.4% in the first month, and it became a light brown liquid and the content was only 20% in the third month.

It can be known from above that the maleate of the present invention has significantly superior stability compared to the formate.

### Example 5

Added 800 g of water, 252 g of sodium bicarbonate, and 270 g of cis-p-methoxycyclohexylaminonitrile maleate into a three-necked flask, and stirred and cooled to 0-10 ° C. 578.4 g of a 30% concentration of 2,5-dimethylphenylacetyl chloride in dichloromethane solution was added dropwise into the above mixture, the temperature was controlled not to exceed 10°C, and a white solid was formed. After the addition was completed, continued stirring and reacting for 2 hours, and then after vacuum filtration, 270 g of the compound of formula II was obtained in the form of a white solid (purity of 98.5% measured by HPLC analysis).

### Example 6

Added 800 g of water, 126 g of sodium carbonate, and 270 g of cis-p-methoxycyclohexylaminonitrile maleate into a three-necked flask, and stirred and cooled to 10-20°C. 578.4 g of a 30% concentration of 2,5-dimethylphenylacetyl chloride in toluene solution was added dropwise into the above mixture, the temperature of the ice-water bath was controlled not to exceed 20°C. After the addition was completed, continued stirring and reacting for 2 hours, and then after vacuum filtration, 265 g of the compound of formula II was obtained in the form of a white solid (purity of 98.0% measured by HPLC analysis).

### Industrial applicability

The method of the present invention can separate cis/trans-p-methoxy cyclohexylaminonitrile with good selectivity to obtain cis-para-substituted cyclohexylaminonitrile salt, which is an important intermediate for the synthesis of spirotetramat.

## Claims

1. The cis-para-substituted cyclohexylaminonitrile salt represented by the following formula (I): 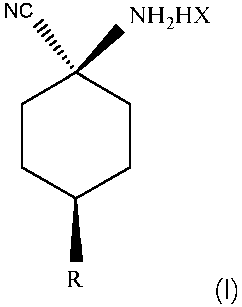 In the above formula (I), the para-substituent R is C₁₋₆ alkyl or alkyloxy, C₁₋₆ alkenyl or alkenyloxy, C₁₋₆ alkynyl or alkynyloxy;
HX is hydroxy-acetic acid or maleic acid.

2. The cis-para-substituted cyclohexylaminonitrile salt of formula (I) according to claim 1, wherein R is methyl or methoxy.

3. A preparation method for the cis-para-substituted cyclohexylaminonitrile salt represented by the above formula (I) according to claim 1, which comprises adding maleic acid or hydroxy-acetic acid into the organic solvent solution of the cis/trans para-substituted cyclohexylaminonitrile compound, stirring and crystallizing, and then separating by filtration or the like to obtain the compound of formula (I).

4. The preparation method according to claim 3, wherein the organic solvent is one selected from methyl tert-butyl ether, ethyl acetate, isopropyl acetate, or a mixture thereof.

5. The preparation method according to claim 3, wherein relative to the cis/trans para-substituted cyclohexylaminonitrile compound, the added amount of maleic acid or hydroxy-acetic acid is 0.3-0.55 mole times, preferably 0.3-0.5 mole times.

6. The preparation method according to claim 3, which further comprises recycling the mother liquor obtained from crystallization and filtration in the preparation method.

7. A method for preparing compound (II), wherein the cis-para-substituted cyclohexylaminonitrile salt of formula (I) according to claim 1 is used as a raw material, the salt compound reacts with 2,5-dimethylphenylacetyl chloride in the presence of a diluent and an acid binding agent to obtain compound (II): 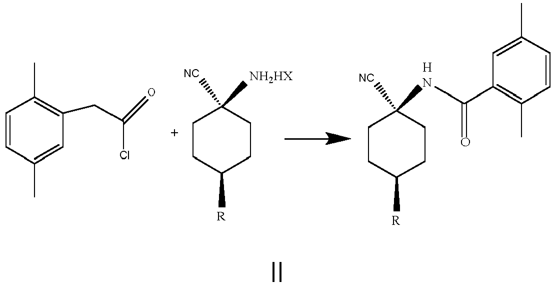 In the above formula, HX and R are as defined in claim 1.

8. The method according to claim 7, wherein the diluent is water and any inert solvent that is not miscible with water.

9. The use of the cis-para-substituted cyclohexylaminonitrile salt of formula (I) according to claim 1 as an intermediate for the preparation of a pesticide.

10. The use according to claim 9, wherein the pesticide is spirotetramat.

## Patentansprüche

1. Ein cis-para-substituierte Cyclohexylaminonitrilsalz, dargestellt durch die folgende Formel (I): 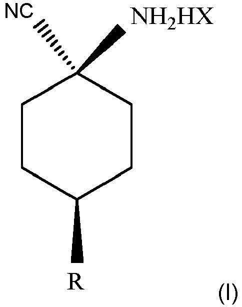 In obiger Formel (I) ist der para-Substituent R C₁₋₆-Alkyl oder Alkyloxy, C₁₋₆-Alkenyl oder Alkenyloxy, C₁₋₆-Alkinyl oder Alkinyloxy;
HX ist Hydroxyessigsäure oder Maleinsäure.

2. Cis-para-substituiertes Cyclohexylaminonitrilsalz der Formel (I) nach Anspruch 1, wobei R Methyl oder Methoxy ist.

3. Ein Verfahren zur Herstellung des cis-para-substituierten Cyclohexylaminonitrilsalzes der obigen Formel (I) nach Anspruch 1, umfassend die Zugabe von Maleinsäure oder Hydroxyessigsäure in die Lösung der cis/trans-para-substituierten Cyclohexylaminonitrilverbindung in einem organischen Lösungsmittel, Rühren und Kristallisieren, und anschließendes Abtrennen durch Filtration oder dergleichen, um die Verbindung der Formel (I) zu erhalten.

4. Herstellungsverfahren nach Anspruch 3, wobei das organische Lösungsmittel ausgewählt ist aus Methyl-tert-butylether, Ethylacetat, Isopropylacetat oder einer Mischung davon.

5. Herstellungsverfahren nach Anspruch 3, wobei bezogen auf die cis/trans-para-substituierte Cyclohexylaminonitrilverbindung die zugesetzte Menge an Maleinsäure oder Hydroxyessigsäure molar das 0,3-0,55-fache, vorzugsweise molar das 0,3-0,5-fache, beträgt.

6. Herstellungsverfahren nach Anspruch 3, das ferner die Rückführung der durch Kristallisation und Filtration gewonnenen Mutterlauge in das Zubereitungsverfahren umfasst.

7. Ein Verfahren zur Herstellung von Verbindung (II), wobei das cis-para-substituierte Cyclohexylaminonitrilsalz der Formel (I) nach Anspruch 1 als Ausgangsmaterial verwendet wird, die Salzverbindung mit 2,5-Dimethylphenylacetylchlorid in Gegenwart eines Verdünnungsmittels und eines Säurebindemittels reagiert, um Verbindung (II) zu erhalten: 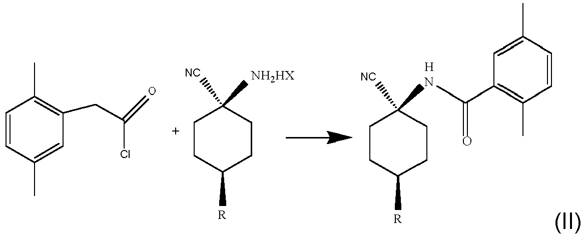 In der obigen Formel sind HX und R wie in Anspruch 1 definiert.

8. Verfahren nach Anspruch 7, wobei das Verdünnungsmittel Wasser und ein beliebiges inertes mit Wasser nicht mischbares Lösungsmittel ist.

9. Die Verwendung des cis-para-substituierten Cyclohexylaminonitrilsalzes der Formel (I) nach Anspruch 1 als Zwischenprodukt zur Herstellung eines Pestizids.

10. Verwendung nach Anspruch 9, wobei das Pestizid Spirotetramat ist.

## Revendications

1. Sel de cyclohexylaminonitrile cis-para-substitué représenté par la formule suivante (I) : 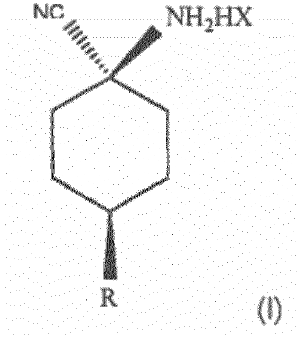 dans la formule (I) ci-dessus, le substituant R en para étant C₁₋₆ alkyle ou alkyloxy, C₁₋₆ alcényle ou alcényloxy, C₁₋₆ alcynyle ou alcynyloxy ;
HX étant l'acide hydroxy-acétique ou l'acide maléique.

2. Sel de cyclohexylaminonitrile cis-para-substitué de formule (I) selon la revendication 1, R étant méthyle ou méthoxy.

3. Procédé de préparation du sel de cyclohexylaminonitrile cis-para-substitué représenté par la formule (I) ci-dessus selon la revendication 1, qui comprend l'ajout d'acide maléique ou d'acide hydroxy-acétique dans la solution de solvant organique du composé de type cyclohexylaminonitrile cis/trans para-substitué, l'agitation et la cristallisation, et ensuite la séparation par filtration ou similaire pour obtenir le composé de formule (I).

4. Procédé de préparation selon la revendication 3, le solvant organique étant l'un choisi parmi le méthyl-tert-butyléther, l'acétate d'éthyle, l'acétate isopropyle ou un mélange correspondant.

5. Procédé de préparation selon la revendication 3, par rapport au composé de type cyclohexylaminonitrile cis/trans para-substitué, la quantité ajoutée d'acide maléique ou d'acide hydroxy-acétique étant de 0,3 à 0,55 fois en moles, préférablement de 0,3 à 0,5 fois en moles.

6. Procédé de préparation selon la revendication 3, qui comprend en outre le recyclage de la liqueur mère obtenue de la cristallisation et de la filtration dans le procédé de préparation.

7. Procédé pour la préparation d'un composé (II), le sel de cyclohexylaminonitrile cis-para-substitué de formule (I) selon la revendication 1 étant utilisé en tant que matière première, le composé de sel réagissant avec le chlorure de 2,5-diméthylphénylacétyle en la présence d'un diluant et d'un agent de liaison d'acide pour obtenir le composé (II) : 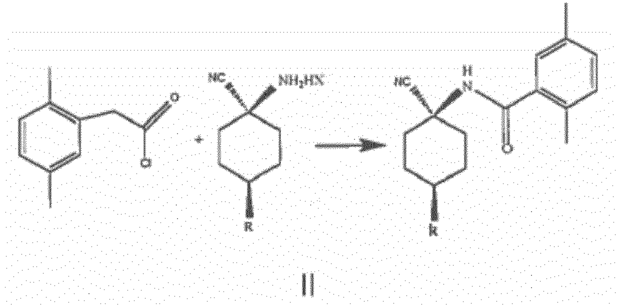 dans la formule ci-dessus, HX et R étant tels que définis dans la revendication 1.

8. Procédé selon la revendication 7, le diluant étant l'eau et un quelconque solvant inerte qui n'est pas miscible à l'eau.

9. Utilisation du sel de cyclohexylaminonitrile cis-para-substitué de formule (I) selon la revendication 1 en tant qu'intermédiaire pour la préparation d'un pesticide.

10. Utilisation selon la revendication 9, le pesticide étant le spirotétramat.
